# EUROPEAN PATENT SPECIFICATION

(11) **EP 1 096 940 B1**
(45) Date of publication and mention of the grant of the patent: **02.04.2003**
(21) Application number: 99932791.9
(22) Date of filing: 02.07.1999
(51) Int. Cl.: A61K 31/728, A61K 9/70, A61P 1/04

(54) **BIOCOMPATIBLE AND BIODEGRADABLE COMPOSITIONS CONTAINING HYALURONIC ACID AND THE DERIVATIVES THEREOF FOR THE TREATMENT OF ULCERS IN THE DIGESTIVE APPARATUS**
BIOLOGISCH KOMPATIBLE UND BIOLOGISCH ABBAUBARE ZUBEREITUNGEN ENTHALTEND HYALURONSÄURE UND DEREN DERIVATE ZUR BEHANDLUNG VON GESCHWÜREN IM VERDAUUNGSAPPARAT
COMPOSITIONS BIOCOMPATIBLES ET BIODEGRADABLES CONTENANT DE L'ACIDE HYALURONIQUE ET DES DERIVES DE CE DERNIER UTILISEES POUR TRAITER LES ULCERES DANS L'APPAREIL DIGESTIF

(30) Priority: 06.07.1998 IT PD980168
(43) Date of publication of application: 09.05.2001
(73) Proprietor: FIDIA ADVANCED BIOPOLYMERS, S.R.L., 35031 Abano Terme (Padova) (IT)
(72) Inventor: CALLEGARO, Lanfranco, I-36016 Thiene (IT); AMBROSIO, Luigi, I-80044 Ottaviano (IT); ESPOSITO, Annaclaudia, I-80123 Napoli (IT)
(74) Representative: Gervasi, Gemma, Dr.
(86) International application number: EP9904604
(87) International publication number: WO00001394

(56) References cited:
- WO-A-93/05792
- WO-A-98/10773
- WO-A-98/48815
- DATABASE WPI Section Ch, Week 9412 Derwent Publications Ltd., London, GB; Class B04, AN 94-097781 XP002116053 & JP 06 048950 A (TAITO KK), 22 February 1994 (1994-02-22)
- DATABASE WPI Section Ch, Week 8616 Derwent Publications Ltd., London, GB; Class B04, AN 86-103236 XP002116054 & JP 61 047418 A (SEIKAGAKU KOGYO CO LTD), 7 March 1986 (1986-03-07)

## Description

### Field of the invention

The present invention concerns the use of hyaluronic acid or the derivatives thereof for the preparation of a matrix as a support for cellular growth for the preparation of biological material to treat ulcers, lesions and diverticula of the digestive and gastrointestinal apparatus.

Said matrix can optionally comprise pharmacologically or biologically active substances. Said matrix comprising cells can be used for the reconstruction of the wall of the digestive apparatus

### Technical Background

Therapy for gastric ulcers has long depended on H2 receptor antagonists such as cimetidine and ranitidine, able to inhibit gastric secretion.

Recently, drugs such as omeprazole which reduce the secretion of gastric acid by a specific mechanism of inhibition of the proton pump at a parietal cell level have been used.

Said drugs are often associated with antibiotics such as amoxacillin, tetracycline, metronidazole and claritromycin that are efficacious against Helicobacter pylori, the gram-negative micro-organism held to be responsible for the recurrence of peptic ulcers.

Surgery is resorted to only in the case of gastric ulcers, suspected to be malignant, or in cases of peptic ulcers complicated by stenosis impairing the passage of food. Cases of perforation also urgently require surgery to close the lesion and avoid potentially fatal consequences such as peritonitis.

Another pathology affecting the digestive system is diverticulitis of the oesophagus.

Diverticula of the digestive tract, particularly the oesophagus, are circumscribed, funnel-shaped or saccate extroversions, that may involve all the layers, or just the mucosa and submucosa of the gut wall.

Food residues stagnating in a diverticulum cause its inflammation, and may also find their way into the respiratory system as a result of coughing or regurgitation, causing bronchitis, bronchial pneumonia and pulmonary abscesses ab ingestis.

Complications such as esophago-bronchial fistulae, oesophagitis, haemorrhages, malignant neoplasia of the diverticulum may also occur.

Treatment of the pathology, must include a suitable diet and the use of antispastic and prokinetic drugs, while more serious cases may require surgery.

Ulcers in the digestive tract may be caused not only by pathologies but also by external trauma, by swallowing sharp foreign bodies or caustic substances.

In the latter case, surgery may prove useless and the affected part may irretrievably lose its functions of absorption, secretion and peristalsis.

The role of hyaluronic acid, a natural polysaccharide, in the process of tissue repair has long been known (Weigel, P. H. et al.: "A model for the role of hyaluronic acid and fibrin in the early events during the inflammatory response and wound healing", *J. Theor. Biol.,* 119: 219, 1986), especially in the early stages of granulation, as it stabilises the coagulation matrix and controls its degradation, favouring the recruitment of inflammatory cells such as polymorphonucleate leukocytes and monocytes, mesenchymal cells such as fibroblasts and endothelial cells, and, lastly, orienting subsequent migration of the epithelial cells.

For the above reasons, hyaluronic acid is widely used in pharmaceutical formulations in the form of creams, sprays and gauzes (Connettivina®) able to accelerate the healing of sores, wounds and burns (EP 0138572).

Moreover, hyaluronic acid derivatives (EP 0216453 B1) are known to be used as scaffolds for the culture of cells such as fibroblasts, keratinocytes, bone marrow stem cells (WO 97/18842) to prepare grafts of bone tissue, cartilage and skin.

Lastly, there are known pharmaceutical preparations in the form of tablets or granules containing esters of acidic polysaccharides with choline (EP 0605478) with antiulcer properties at a gastric level due to their ability to form gels and protect the mucosa.

The Japanese Patent No. JP 6048950 relates to antiulcer agents containing as the active principle at least one zinc salt of low molecular weight compound of acidic polysaccharides, including hyaluronic acid.

The Japanese Patent No. JP 61047418 discloses a pharmaceutical composition for protecting stomach mucosa containing hyaluronic acid or a salt thereof, lecithin and protein.

WO 98/10773 relates to pharmaceutical compositions having antimicrobial activity containing as the active ingredient associates (complexes) of hyaluronic acid, i.e. zinc and cobalt hyaluronates.

The European Patent No. EP 526 756 describes a pharmaceutical composition for revitalising scar tissue comprising a bioactive material selected from naturally occurring growth factors, active peptide segments of various growth factors, synthetic growth factors and admixtures thereof; and additionally comprising a pharmaceutically acceptable controlled release matrix, including hyaluronic acid, wherein the bioactive material is immobilised

Although hyaluronic acid and its derivatives are used topically, their application in the digestive and gastrointestinal systems is as yet unknown.

It has now been discovered, surprisingly, that compositions based on hyaluronic acid or its derivatives, in association with growth factors or cell cultures, suitable for endoscopic administration, can be used effectively in the treatment of lesions or ulcers in the digestive and gastrointestinal systems.

Where there is loss or degeneration of a large area of tissue or perforation of the wall of the digestive apparatus, surgery can be performed and it is possible to reconstruct the injured part by grafting cell cultures grown on scaffolds constituted by hyaluronic acid derivatives.

### Summary of the invention

The Applicant has unexpectedly found that hyaluronic acid and derivatives thereof may be advantageously used in the treatment of ulcers, lesions and diverticula of the digestive and gastrointestinal apparatus.

The present invention therefore relates to the use of hyaluronic acid or a derivative thereof for the preparation of a matrix as a support for cellular growth for the preparation of biological material for the treatment of the above mentioned diseases.

### Brief description of the drawings

Figure 1 shows electron microscope image (Mag: 14.97 K X) on the 38^{th} day of culture grown on Petri dishes;
Figure 2 shows electron microscope image (Mag: 17.50 K X) on the 38^{th} day of culture grown on transwells;
Figure 3 shows electron microscope image (Mag: 17.50 K X) on the 38^{th} day of culture grown on Laserskin® (bidimensional matrix comprising hyaluronic acid esters);
Figure 4 shows electron microscope image (Mag: 15.02 K X) on the 38^{th} day of culture grown on Hyaff11 3D (three-dimensional matrix comprising hyaluronic acid esters);
Figure 4a shows electron microscope image (Mag: 898 X) on the 38^{th} day of culture grown on Hyaff11 3D (three-dimensional matrix comprising hyaluronic acid esters);
Figure 5 shows electron microscope image (Mag: 15.02 K X) on the 38^{th} day of culture grown on polyurethane;
Figure 6 shows diagrams with in ordinates ALP (alkaline phosphates) Activity (mU/mg of proteins) and in abscissae days of culture.

### Detailed description of the invention

Of the hyaluronic acid derivatives that can be used according to the present invention the following are to be preferred:
- hyaluronic acid esters wherein part or all of the carboxy functions are esterified with alcohols of the aliphatic, aromatic, arylaliphatic, cycloaliphatic, heterocyclic series (EP 0216453 B1);
- autocross-linked esters of hyaluronic acid wherein part or all of the carboxy groups are esterified with the alcoholic functions of the same polysaccharide chain or other chains (EP 0341745 B1);
- cross-linked hyaluronic acid compounds wherein part or all of the carboxy groups are esterified with polyalcohols of the aliphatic, aromatic, arylaliphatic, cycloaliphatic or heterocyclic series, generating cross-linking by means of spacer chains (EP 0265116 B1);
- hemiesters of succinic acid or the heavy metal salts of the hemiester of succinic acid with hyaluronic acid or with partial or total esters of hyaluronic acid (WO 96/357207);
- O-sulphated derivatives (WO 95/25751) or N-sulphated derivatives (WO 98/45335);
- amidic derivatives of hyaluronic acid or of the compounds listed above obtained by reaction of a primary or secondary amine of the aliphatic, aromatic, arylaliphatic, cycloaliphatic or heterocyclic series, that can optionally be a pharmaceutically active substance, with a free carboxylic group of hyaluronic acid or a derivative thereof; or by reaction of an acid of the aliphatic, aromatic, arylaliphatic or cycloaliphatic series, that can optionally be a pharmaceutically active substance, with a deacylated amino group of hyaluronic acid or a derivative thereof.

The matrix according to the present invention may also contain pharmacologically or biologically active substances such as antibiotics, in particular antibiotics active against Helicobacter pylori, growth factors, antimicotics, antimicrobials and antiviral agents. Compositions containing antibiotics active against Helicobacter pylori are for example in the form of mixtures or salts, or covalently bound with the aforesaid hyaluronic acid derivatives; heavy metal salts such as zinc and cobalt, salts of the hemiester of succinic acid or hyaluronic acid or with partial or total esters of hyaluronic acid.

The matrix used according to the present invention is preferably in the form of non-woven fabric, or perforated membranes.

Bidimensional or three dimensional matrix containing a hyaluronic acid derivative, may be used as support for cellular growth for the preparation of biological material containing suitable cell cultures for regenerating the walls or filling diverticula in the digestive apparatus. Said cells can be mature intestinal cells, mesenchymal cells, fibroblasts, epithelial cells or mixture thereof.

These biological materials for example may contain intestinal cells useful in the reconstruction of injured digestive apparatus. These biological materials are implanted onto the lesion site by surgical methods.

### EXAMPLE

### Growth of epithelial cells on scaffolds made of benzyl esters of hyaluronic acid

Intestinal cells were seeded onto scaffolds made of the total benzyl ester of hyaluronic acid in the form of a perforated membrane and non-woven fabric, in order to test their biocompatibility, and their morphological and biochemical responses were observed.

The cells belonged to the CaCO₂ cell line (derived from human colon carcinoma) that differentiate spontaneously into enterocytes typical of the mature intestinal epithelium.

The cells were used at passage 98. They were seeded at a density of about 9 x 10³/cm² in DMEM 4.5 g of glucose/L containing 20% FBS penicillin/streptomycin, fungizone and non-essential amino acids (1%) in a humidified atmosphere with 95% CO₂. The culture medium was changed every 48 hours. Other cells were seeded on Petri dishes and Transwell wells with polycarbonate membranes in the same culture conditions and served as controls.

Polyurethane (chronoflex_TM), a material for biomedical purposes, was used as negative control. On the 3^{rd}, 15^{th}, 20^{th} and 40^{th} days of culture, the cells were prepared for observation user scanning electron microscope (SEM) and for assessment of the total proteins and the activity of alkaline phosphates (ALP) according to the following methods: SEM fixing in 2.5% glutaraldehyde in phosphate buffer (PBS) pH 7.4. Osmium tetroxide, 1% in PBS, dehydration in ethanol and increasing concentrations of up to 100% and dehydration with a Critical Point drier. The cells were then metalized with gold and observed by SEM. ALP activity: the cells were harvested by scraping in a lysis buffer 2mM Tris-HCl 50 mM mannitol pH 7.2 (1 ml final volume) (with the exception of those seeded on Hyaff 3D) and sonicated in ice. ALP activity of the cellular lysates was determined by spectrophotometry by hydrolysis of the p-nitrophenylphosphate using a Boehringer kit. The total proteins were determined by Lowry's method. The activity present in the cells grown on a scaffold in the form of a non-woven fabric was determined in lysates obtained by sonicating the membrane containing the cells *in toto.*

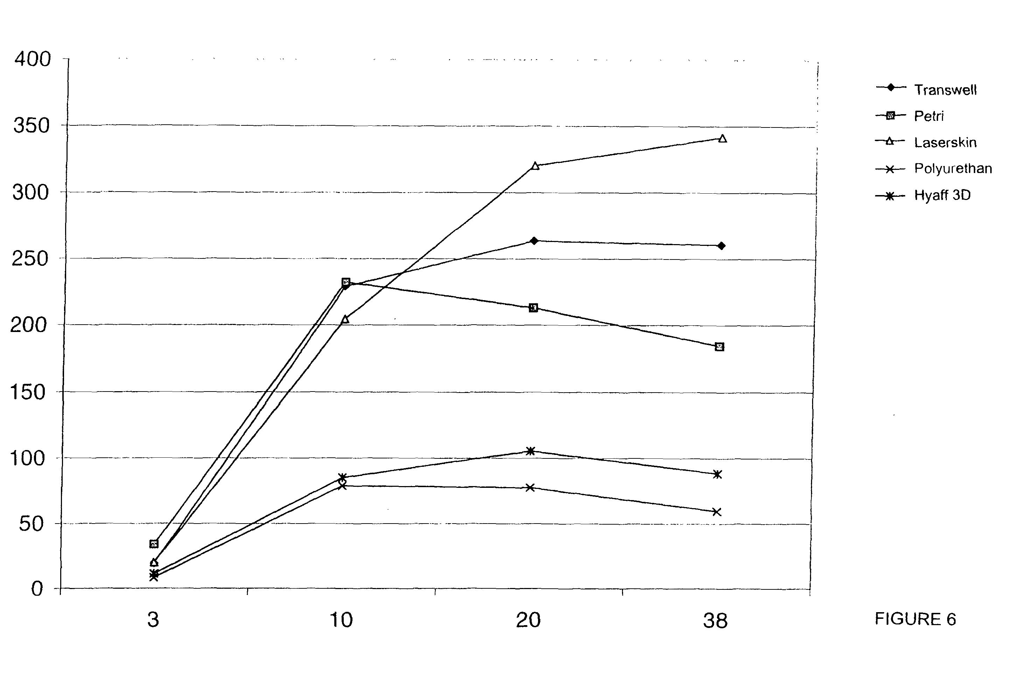

Morphological differentiation was assessed on the basis of the presence of microvilli on the upper surface of the cells, while the biochemical differentiation was assessed on the basis of the increase of ALP activity (see results in Figure 6).

Both were considered as biocompatibility parameters.

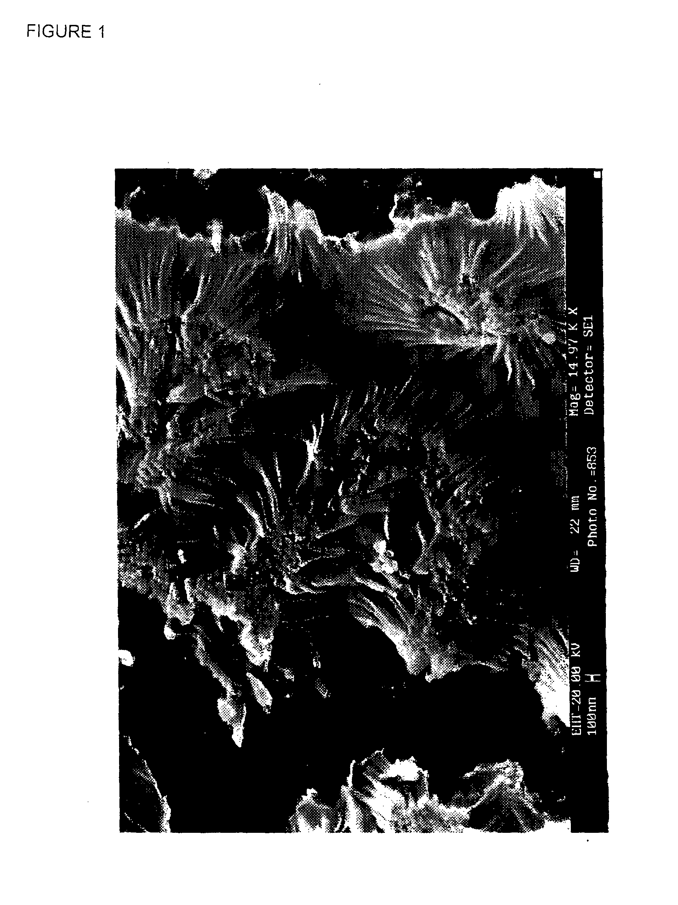

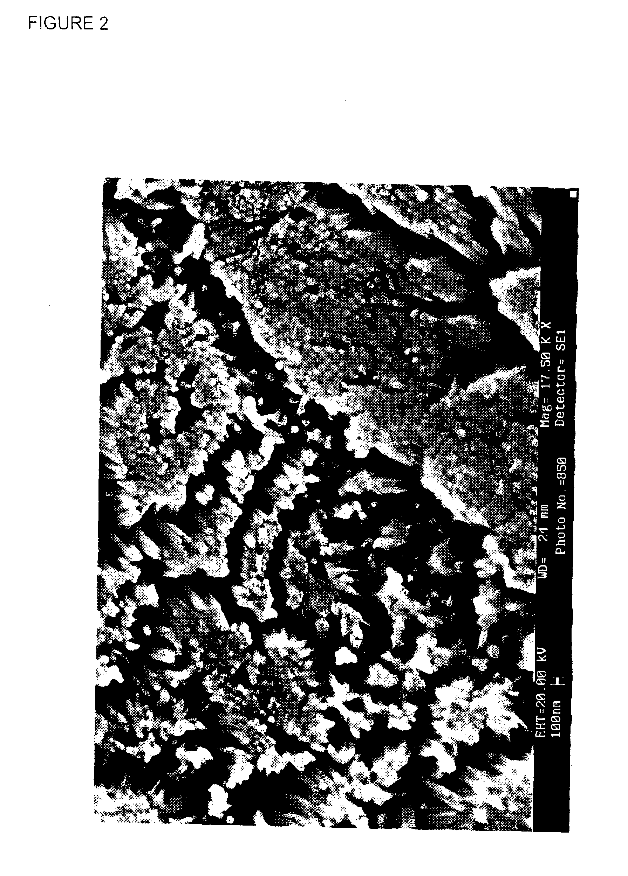

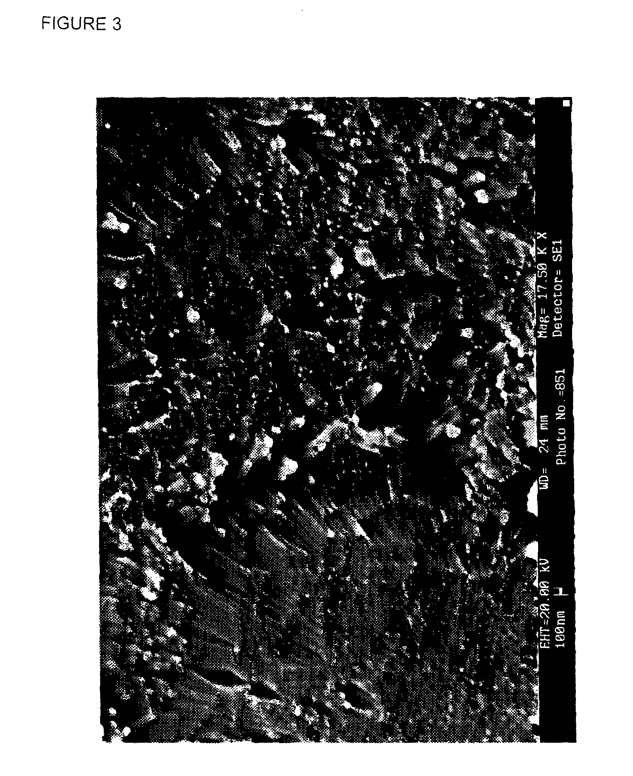

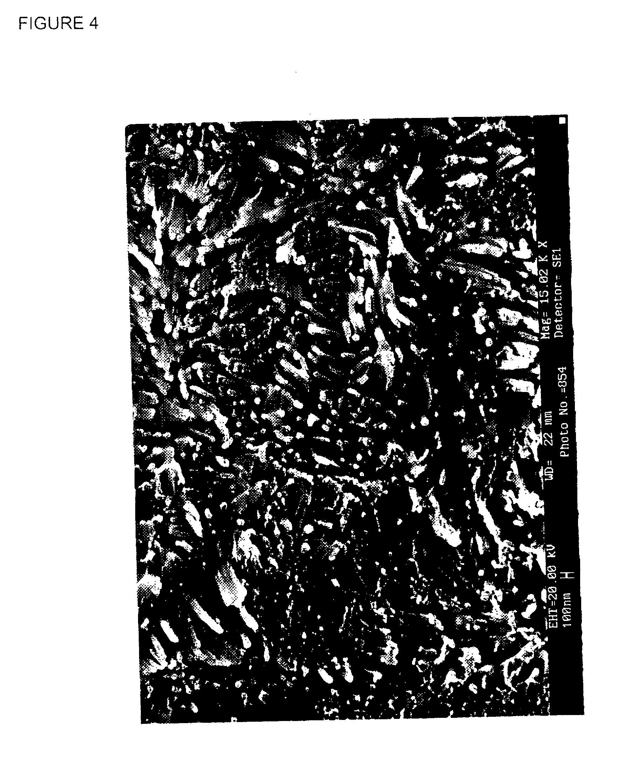

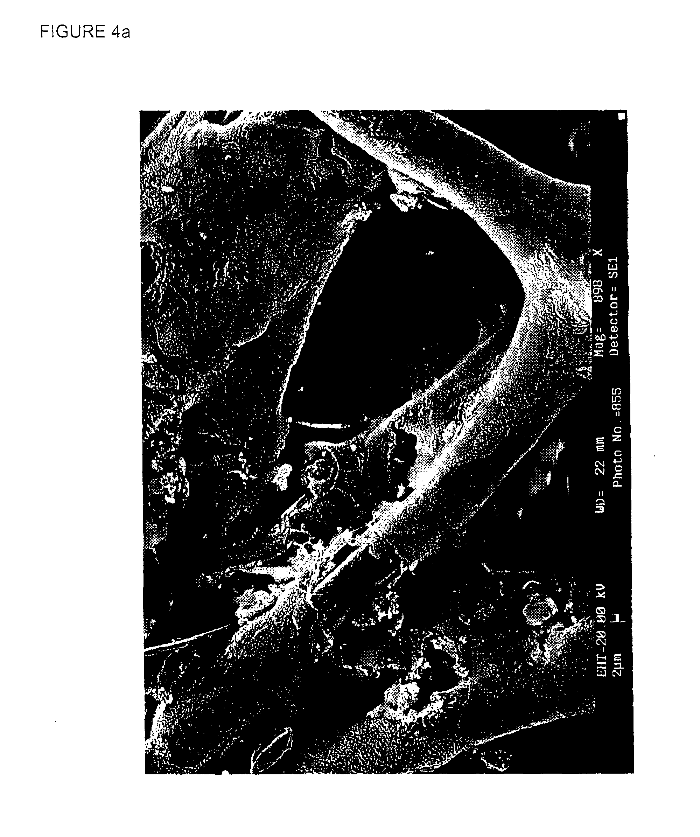

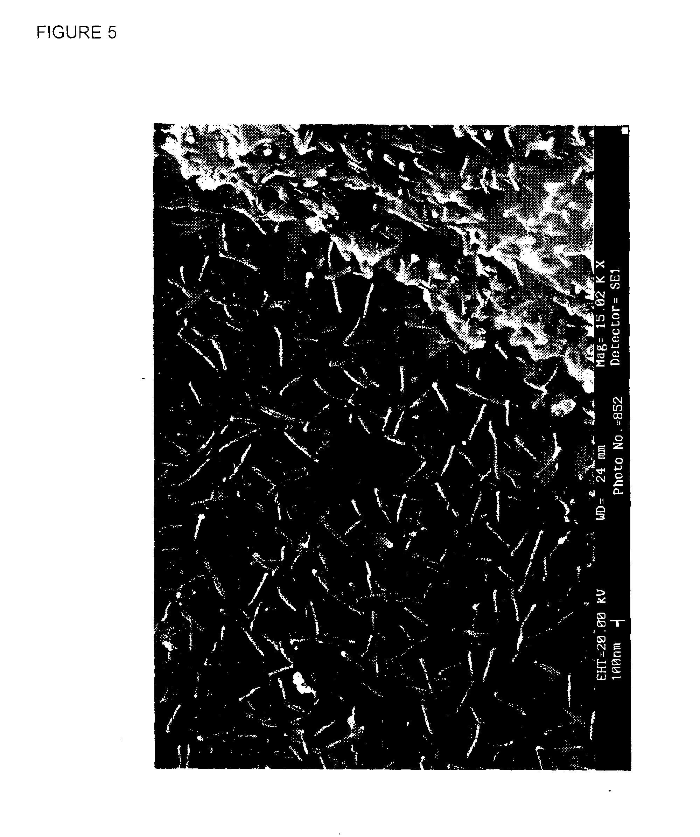

Figures 1, 2, 3, 4, 4a and 5 show electron microscope images of the cells on the 38^{th} day of culture, grown on Petri dishes, transwells, membranes of hyaluronic acid (Laserskin®), hyaluronic acid matrices (Hyaff11 3D) and polyurethane membranes respectively. As can be seen, the cells grown on Laserskin and Transwell show marked differentiation due to the appearance of numerous microvilli on their surfaces, whereas those grown on Petri dishes show fewer, less well developed microvilli. The cell grown on the scaffold (in the form of a non-woven fabric) and Chronoflex do not show any formation of microvilli, while those grown on Chronoflex alone present extroversion indicative of cell suffering.

## Claims

1. Use of a matrix comprising at least one hyaluronic acid or a derivative thereof, for the preparation of a biological material for the treatment of ulcers, lesions and diverticula of the digestive and gastrointestinal apparatus, wherein said matrix is a scaffold for cellular growth, and said biological material is implantable onto the lesion site by surgery.

2. The use according to claim 1, wherein the hyaluronic acid derivatives are hyaluronic acid esters wherein part or all of the carboxy functions are esterified with alcohols of the aliphatic, aromatic, arylaliphatic, cycloaliphatic, heterocyclic series.

3. The use according to claim 1, wherein the hyaluronic acid derivatives are the cross-linked esters of hyaluronic acid wherein part or all of the carboxy groups are esterified with the alcoholic functions of the same polysaccharide chain or other chains.

4. The use according to claim 1, wherein the hyaluronic acid derivatives are the cross-linked compounds of hyaluronic acid wherein part or all of the carboxy groups are esterified with polyalcohols of the aliphatic, aromatic, arylaliphatic, cycloaliphatic, heterocyclic series, generating cross-linking by means of spacer chains.

5. The use according to claim 1, wherein the hyaluronic acid derivatives are hemiesters of succinic acid or heavy metal salts of the hemiester of succinic acid with hyaluronic acid or partial or total esters of hyaluronic acid.

6. The use according to claim 1, wherein the hyaluronic acid derivatives are O-sulphated or N-sulphated hyaluronic acid derivatives.

7. The use according to claim 1, wherein the hyaluronic acid derivatives are hyaluronic acid amides wherein part or all the free carboxylic groups of hyaluronic acid are reacted with a primary or a secondary amine chosen from the group consisting of the aliphatic, aromatic, arylaliphatic, cycloaliphatic or heterocyclic amine, that can optionally be a pharmaceutically active substance.

8. The use according to claim 1, wherein the hyaluronic acid derivatives are amide wherein a deacylated amino group of hyaluronic acid or of a derivative thereof as defined in claims 2-6, is reacted with an acid chosen from the group consisting of the aliphatic, aromatic, arylaliphatic or cycloaliphatic acid, that can optionally be a pharmaceutically active substance.

9. The use according to claim 1, wherein the said matrix is in the form of a non woven fabric.

10. The use according to claim 1, wherein the said matrix is in the form of a perforated membrane.

11. The use according to claim 1, wherein the cells are chosen from the group consisting of mature cells, mesenchimal cells, fibroblasts, epithelial cells and mixtures thereof.

12. A biological material comprising:
a) intestinal cells optionally together with fibroblasts, mesenchimal cells, mature cells and/or epithelial cells;
b) a matrix comprising at least one hyaluronic acid derivative as defined in claims 2-8,
wherein said matrix is a scaffold on which said cells are grown, and said biologic material is implantable by surgery onto the lesion site.

13. The biological material according to claim 12, wherein said matrix is in the form of a non woven tissue.

14. The biological material according to claim 12, wherein said matrix is in the form of a perforated membrane.

## Patentansprüche

1. Verwendung einer Matrix, die wenigstens eine Hyaluronsäure oder ein Derivat davon umfasst, zur Herstellung eines biologischen Materials zur Behandlung von Geschwüren, Läsionen und Divertikeln des Verdauungs- und Magen-Darm-Trakts, worin die Matrix ein Gerüst für Zellwachstum ist und das biologische Material auf die Läsion durch Operation implantierbar ist.

2. Verwendung gemäss Anspruch 1, worin die Hyaluronsäurederivate Hyaluronsäureester sind, worin ein Teil oder alle Carboxyfunktionen mit Alkoholen der aliphatischen, aromatischen, arylaliphatischen, cycloaliphatischen oder heterocyclischen Reihe verestert sind.

3. Verwendung gemäss Anspruch 1, worin die Hyaluronsäurederivate die vernetzten Ester von Hyaluronsäure sind, worin ein Teil oder alle Carboxygruppen mit den alkoholischen Funktionen der gleichen Polysaccharidkette oder anderer Ketten verestert sind.

4. Verwendung gemäss Anspruch 1, worin die Hyaluronsäurederivate die vernetzten Verbindungen von Hyaluronsäure sind, worin ein Teil oder alle Carboxygruppen mit Polyalkoholen der aliphatischen, aromatischen, arylaliphatischen, cycloaliphatischen oder heterocyclischen Reihe verestert sind, wobei die Vernetzung mittels Spacer-Ketten erzeugt wird.

5. Verwendung gemäss Anspruch 1, worin die Hyaluronsäurederivate Halbester der Bernsteinsäure oder Schwermetallsalze des Halbesters der Bernsteinsäure mit Hyaluronsäure oder Partial- oder Vollester von Hyaluronsäure sind.

6. Verwendung gemäss Anspruch 1, worin die Hyaluronsäurederivate O-sulfatierte oder N-sulfatierte Hyaluronsäurederivate sind.

7. Verwendung gemäss Anspruch 1, worin die Hyaluronsäurederivate Hyaluronsäureamide sind, worin ein Teil oder alle freien Carbonsäuregruppen der Hyaluronsäure mit einem primären oder sekundären Amin umgesetzt sind, das aus der Gruppe ausgewählt ist, die aus einem aliphatischen, aromatischen, arylaliphatischen, cycloaliphatischen oder heterocyclischen Amin besteht, das gegebenenfalls eine pharmazeutisch aktive Substanz sein kann.

8. Verwendung gemäss Anspruch 1, worin die Hyaluronsäurederivate Amide sind, worin eine desacylierte Aminogruppe von Hyaluronsäure oder eines Derivats davon wie in den Ansprüchen 2 bis 6 definiert mit einer Säure umgesetzt ist, die aus der Gruppe ausgewählt ist, die aus einer aliphatischen, aromatischen, arylaliphatischen oder cycloaliphatischen Säure besteht, die gegebenenfalls eine pharmazeutisch aktive Substanz sein kann.

9. Verwendung gemäss Anspruch 1, worin die Matrix in Form eines Vliesgewebes ist.

10. Verwendung gemäss Anspruch 1, worin die Matrix in Form einer perforierten Membran ist.

11. Verwendung gemäss Anspruch 1, worin die Zellen aus der Gruppe ausgewählt sind, die aus reifen Zellen, Mesenchymzellen, Fibroblasten, Epithelzellen und Mischungen daraus besteht.

12. Biologisches Material, umfassend:
(a) Darmzellen, gegebenenfalls zusammen mit Fibroblasten, Mesenchymzellen, reifen Zellen und/oder Epithelzellen;
(b) eine Matrix, die wenigstens ein Hyaluronsäurederivat wie in den Ansprüchen 2 bis 8 definiert umfasst,
worin die Matrix ein Gerüst ist, auf dem die Zellen wachsen, und das biologische Material durch Operation auf die Läsionsstelle implantierbar ist.

13. Biologisches Material gemäss Anspruch 12, worin die Matrix in Form eines Vliesgewebes ist.

14. Biologisches Material gemäss Anspruch 12, worin die Matrix in Form einer perforierten Membran ist.

## Revendications

1. Utilisation d'une matrice comprenant au moins de l'acide hyaluronique ou au moins un dérivé de celui-ci, pour la préparation d'un matériau biologique pour le traitement d'ulcères, de lésions et de diverticules de l'appareil digestif et gastro-intestinal, dans laquelle ladite matrice constitue un accrochage pour la croissance cellulaire, et ledit matériau biologique peut être implanté sur le site de la lésion par un acte chirurgical.

2. Utilisation suivant la revendication 1, dans laquelle les dérivés d'acide hyaluronique sont des esters d'acide hyaluronique dans lesquels une partie ou la totalité des fonctions carboxyliques sont estérifiées avec des alcools des séries aliphatique, aromatique, arylaliphatique, cycloaliphatique, hétérocyclique.

3. Utilisation suivant la revendication 1, dans laquelle les dérivés d'acide hyaluronique sont les esters réticulés d'acide hyaluronique dans lesquels une partie ou la totalité des groupes carboxy sont estérifiées avec les fonctions alcooliques de la même chaîne polysaccharidique ou d'autres chaînes.

4. Utilisation suivant la revendication 1, dans laquelle les dérivés d'acide hyaluronique sont les composés réticulés d'acide hyaluronique dans lesquels une partie ou la totalité des groupes carboxy sont estérifiées avec des polyalcools des séries aliphatique, aromatique, arylaliphatique, cycloaliphatique, hétérocyclique, ce qui génère des réticulations par des chaînes d'espacement.

5. Utilisation suivant la revendication 1, dans laquelle les dérivés d'acide hyaluronique sont des hémiesters d'acide succinique ou de sels de métaux lourds de l'hémiester d'acide succinique avec l'acide hyaluronique ou des esters partiels ou totaux d'acide hyaluronique.

6. Utilisation suivant la revendication 1, dans laquelle les dérivés d'acide hyaluronique sont des dérivés d'acide hyaluronique 0-sulfatés ou N-sulfatés.

7. Utilisation suivant la revendication 1, dans laquelle les dérivés d'acide hyaluronique sont des amides d'acide hyaluronique dans lesquels une partie ou la totalité des groupes carboxyliques libres d'acide hyaluronique sont traitées avec une amine primaire ou secondaire choisie dans le groupe consistant en amine aliphatique, aromatique, arylaliphatique, cycloaliphatique ou hétérocyclique, qui peut éventuellement être une substance active du point de vue pharmaceutique.

8. Utilisation suivant la revendication 1, dans laquelle les dérivés d'acide hyaluronique sont un amide dans lequel un groupe amino désacylé d'acide hyaluronique ou d'un dérivé de celui-ci suivant les revendications 2 à 6, est traité avec un acide choisi dans le groupe consistant en acide allphatique, aromatique, arylaliphatique ou cycloaliphatique, qui peut éventuellement être une substance active du point de vue pharmaceutique.

9. Utilisation suivant la revendication 1, dans laquelle ladite matrice est sous la forme d'un tissu non tissé.

10. Utilisation suivant la revendication 1, dans laquelle ladite matrice est sous la forme d'une membrane perforée.

11. Utilisation suivant la revendication 1, dans laquelle les cellules sont choisies dans le groupe consistant en cellules matures, cellules de mésenchyme, fibroblastes, cellules épithéliales et leurs mélanges.

12. Matériau biologique comprenant :
a) des cellules intestinales éventuellement avec des fibroblastes, des cellules de mésenchyme, des cellules matures et/ou des cellules épithéliales ;
b) une matrice comprenant au moins un dérivé d'acide hyaluronique suivant les revendications 2 à 8,
dans lequel ladite matrice est un accrochage sur lequel lesdites cellules sont cultivées, et ledit matériau biologique peut être implanté par un acte chirurgical sur le site de la lésion.

13. Matériau biologique suivant la revendication 12, dans lequel ladite matrice est sous la forme d'un tissu non tissé.

14. Matériau biologique suivant la revendication 12, dans lequel ladite matrice est sous la forme d'une membrane perforée.
